# EUROPÄISCHE PATENTANMELDUNG

(11) **EP 2 695 599 A1**
(43) Veröffentlichungstag der Anmeldung: **12.02.2014**
(21) Anmeldenummer: 13177372.3
(22) Anmeldetag: 22.07.2013
(51) Int. Cl.: A61K 8/42, A61Q 19/00, A61K 8/97, A61K 8/35, A61K 8/06

(54) **Kosmetische oder dermatologische Zubereitungen mit einem Gehalt an Harnstoff und einem Gehalt an Licochalcon A oder einem wäßrigen Extrakt aus Radix Glycyrrhizae inflatae, enthaltend Licochalcon A**

(30) Priorität: 07.08.2012 DE 102012213935
(71) Anmelder: Beiersdorf AG, 20253 Hamburg (DE)
(72) Erfinder: Filbry, Alexander, 22459 Hamburg (DE); Kröpke, Rainer, 22869 Schenefeld (DE); Ruhs, Joanna, 22043 Hamburg (DE)

(57) **Zusammenfassung**

Kosmetische oder dermatologische Zubereitungen, insbesondere in Form von Wasser- in -Öl-Emulsionen, mit einem Gehalt an
(a) Harnstoff
(b) Licochalcon A oder einem wäßrigen Extrakt aus *Radix Glycyrrhizae inflatae,* enthaltend Licochalcon A.

## Beschreibung

Die vorliegende Erfindung betrifft kosmetische oder dermatologische Zubereitungen mit einem Gehalt an Harnstoff und einem Gehalt an Licochalcon A oder einem wäßrigen Extrakt aus Radix Glycyrrhizae inflatae, enthaltend Licochalcon A.

Harnstoff gilt in ca. 2%igen Zubereitungen als *granulationsfördernd* (als Granulationsgewebe bezeichnet man das typische junge, gefäßreiche Bindegewebe, das bei der Wund- und Geschwürheilung bzw. bei chronischen Entzündungen gebildet wird und sich nach einiger Zeit in Narbengewebe umbildet).

In höheren Konzentrationen (5-10%) nutzt der Hautarzt den *hygroskopischen* (wasseranziehenden) Effekt des Harnstoffs aus, z.B. bei der Behandlung der Ichthyosis ("Fischschuppenkrankheit"), der Schuppenflechte (Psoriasis) und der Neurodermitis (atopisches Ekzem). *Hygroskopische* Substanzen, in erster Linie Harnstoff, halten als natürliche Feuchthaltefaktoren die Feuchtigkeit in der Hornhaut zurück. Sinkt der Wassergehalt in der Hornschicht (verursacht durch einen Harnstoffmangel) unter 15%, z.B. bei der Neurodermitis atopica, wird die Haut trocken, spröde und rissig. Ähnlich wie beim atopischen Ekzem fehlt auch bei der Schuppenflechte Harnstoff in der Epidermis. Die Folge ist eine starke Austrocknung der befallenen Hautflächen.

In kosmetischen Hautpräparaten verwendet man Harnstoff als in der Haut feuchtigkeitsbindende Substanz (1 bis 2%ig). Eine 10%ige wäßrige Lösung von Harnstoff ist bakterientötend. Schließlich wirkt Harnstoff - was man sich sowohl in Pharmazie wie auch Kosmetik zunutze machen kann - keratolytisch bzw. keratoplastisch (erweichender Effekt auf das Keratin der Haut) sowie juckreizmildernd. Durch seine polare Struktur hält er das Wasser in der Haut fest und bewahrt ihr dadurch Glätte und Geschmeidigkeit.

Die Wirkung des Wirkstoffs Allantoin beruht ebenfalls auf Harnstoff, da dieser aus dem Allantoin abgespalten wird.

Problematisch ist aber, daß gerade natürliche Öle in Kombination mit Harnstoff schwer zu formulieren sind.

Für die Entwicklung stabilier Wasser-in-Öl-Emulsionen mußte daher bisher immer auf ethoxylierte Emulgatoren zurückgegriffen werden. Will man eine angenehme Sensorik des Produktes erreichen, mußte man dem Produkt infolgedessen oft Sensorikadditive zusetzen, weil ethoxylierte Verbindungen dazu neigen, der kosmetischen Formulierung eine gewisse Klebrigkeit zu verleihen.

Ein weiteres Problem war es, daß dergleichen Zubereitungen sich oft durch eine niedrige Stabilität auszeichnen, insbesondere, wenn sie als Lotionen, also niedirgviskose Zubereitungen vorliegen sollten.

Aufgabe der vorliegenden Erfindung war es, die Nachteile des Standes der Technik zu beseitigen.

Überraschenderweise wird die Aufgabe gelöst durch
kosmetische oder dermatologische Zubereitungen, insbesondere in Form von Wasser - in -Öl-Emulsionen, mit einem Gehalt an
(a) Harnstoff
(b) Licochalcon A oder einem wäßrigen Extrakt aus *Radix Glycyrrhizae inflatae,* enthaltend Licochalcon A

Die Pflanzenart *Glycyrrhiza inflata* gehört wie das in Europa offizinelle Süßholz *Glycyrrhiza glabra* der Gattung *Glycyrrhiza* an, die zur Pflanzenfamilie der *Fabaceae* (Erbsengewächse) gehört. Die Droge *Radix Glycyrrhizae inflatae,* d.h. die Wurzel der Pflanze, ist beispielsweise in der fernöstlichen Medizin gebräuchlich. Die Verwendung der Droge als Entzündungshemmer ist ebenfalls bekannt.

Ein Bestandteil des wäßrigen Auszugs aus Radix Glycyrrhizae inflatae ist das Licochalcon A, welches sich durch die folgende Strukturformel auszeichnet:

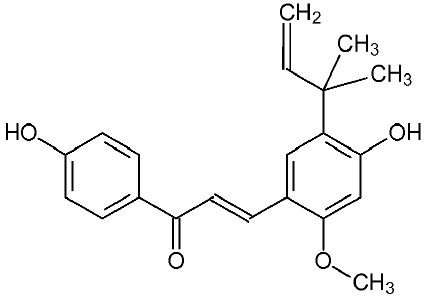

Es wird angenommen, daß diese Substanz, möglicherweise in Synergie mit den übrigen Bestandteilen des Extraktes, Anteil an der erfindungsgemäßen Wirkung besitzt.

Vorteilhaft ist erfindungsgemäß insbesondere, wenn die Zubereitungen 0,0001 bis 5 Gew.-%, insbesondere 0,001 bis 1 Gew.-%, ganz besonders 0,005 bis 0,15 Gew.-% Licochalcon A enthalten, bezogen auf das Gesamtgewicht der Zubereitung.

Vorteilhaft ist erfindungsgemäß ferner insbesondere, wenn die Zubereitungen 0,001 bis 20 Gew.-%, insbesondere 0,05 bis 10 Gew.-%, ganz besonders 0,01 bis 5 Gew.-% an einem oder mehreren Polyolen enthalten, bezogen auf das Gesamtgewicht der Zubereitung.

Verwendungsgemäße Polyole sind Glycerin, Butylenglycol, Dipropylenglycol, Propylenglycol, Pentandiol, Hexandiol.

Vorteilhaft ist erfindungsgemäß ferner wenn die Zubereitungen Licocalchon als Bestandteil von pflanzlichen Extracten, insbesondere von Radix Glycyrrhizae inflatae, enthalten.

Erfindungsgemäß ist ferner vorteilhaft, wenn Licochalcon in Form eines wäßrigen Auszugs vorliegt, in welchem
- Licochalcon A
- Wasser
- gegebenenfalls ein oder mehrere Polyole
vorliegen.

Es ist erfindungsgemäß vorteilhaft, wenn die kosmetischen oder dermatologischen Zubereitungen 0,0001 bis 10 Gew.-%, insbesondere 0,005 bis 5 Gew.-%, ganz besonders 0,01 bis 1 Gew.-% an einem wäßrigen Extrakt aus Radix Glycyrrhizae inflatae enthalten, bezogen auf das Gesamtgewicht der Zubereitung.

Es ist erfindungsgemäß vorteilhaft, wenn die kosmetischen oder dermatologischen Zubereitungen 0,001 bis 20 Gew.-%, insbesondere 0,05 bis 10 Gew.-%, ganz besonders 0,01 bis 5 Gew.-% an einem oder mehreren Polyolen enthalten, bezogen auf das Gesamtgewicht der Zubereitung.

Insbesondere ist vorteilhaft, als Polyol das Butylenglycol zu wählen.

Ganz besonders vorteilhaft ist es, von einem Extrakt auszugehen, der unter der Bezeichnung Polyol Soluble Licorice Extract P-U von der Firma Maruzen vertrieben wird.

Ferner ist es vorteilhaft, Licochalcone A in anderen Vehikelsystemen in einer Konzentration von 0,0001 bis 5 Gew.-%, insbesondere 0,001 bis 1 Gew.-%, ganz besonders 0,005 - 0,05 Gew.-% zu verwenden.

Kosmetische oder dermatologische Zubereitungen gemäß der Erfindung enthalten vorteilhaft 0,01 bis 60 Gew.-%, bevorzugt 0,1 bis 25 Gew.-%, insbesondere bevorzugt 1 bis 10 Gew.-% an Harnstoff, bezogen auf das Gesamtgewicht der Zubereitungen.

Kosmetische oder dermatologische Zubereitungen gemäß der Erfindung enthalten vorteilhaft 0,0001 bis 15 Gew.-%, bevorzugt 0,01 bis 10 Gew.-%, insbesondere bevorzugt 0,05 bis 2,5 Gew.-% an Polyglyceryl-4 Diisostearat/Polyhydroxystearate/Sebacat, bezogen auf das Gesamtgewicht der Zubereitungen.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Die kosmetischen und dermatologischen Zubereitungen gemäß der Erfindung können kosmetische oder galenische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate. Ein zusätzlicher Gehalt an üblichen Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1-10 Gew.-%, bezogen auf das Gesamtgewicht der Zubereitung.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, Glycerylglucose, NMF-Komplex, Ceramide, Cholesterol, Sterol oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen im Sinne der vorliegenden Erfindung sind die üblichen bekannten leicht flüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder - distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wäßrigen Phase einzusetzen.

Vorteilhaft können die Zubereitungen erfindungsgemäß weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Kosmetika mit Harnstoff und einem Gehalt an Licochalcone A**

| | **1** | **2** | **3** | **4** | **5** |
|---|---|---|---|---|---|
| Arginin | 0,5 | 0,5 | 0,5 | 0,5 | 0,5 |
| Hydroxyethylcellulose | 0,1 | 0,15 | 0,25 | - | 1,0 |
| Cetrimonium Chlorid | 0,1 | 0,1 | - | - | 0,1 |
| Glycerin | 0,5 | 1,0 | - | 1,5 | 1,5 |
| Harnstoff | 3,0 | 5,0 | 2,5 | 1,0 | 10,0 |
| Milchsäure | 4,0 | 4,0 | 4,0 | 4,0 | 4,0 |
| Glycyrrhiza Inflata Extrakt enthaltend Licochalcon A | 0,01 | 0,025 | 0,05 | 0,075 | 1,0 |
| Methylpropandiol | - | 2,0 | 2,0 | 2,0 | 2,0 |
| Natriumlaktat | 4,0 | 4,0 | 4,0 | 4,0 | 4,0 |
| PEG-40 Hydriertes Rizinusöl | - | 0,1 | 0,1 | 0,1 | - |
| Phenoxyethanol | 0,6 | 0,6 | 0,6 | 0,4 | 0,6 |
| Polydocanol | 2,0 | 2,0 | 2,0 | 2,0 | - |
| Wasser | Ad 100 | Ad 100 | Ad 100 | Ad 100 | Ad 100 |

## Patentansprüche

1. Kosmetische oder dermatologische Zubereitungen, insbesondere in Form von Wasser - in - Öl-Emulsionen, mit einem Gehalt an
(a) Harnstoff
(b) Licochalcon A oder einem wäßrigen Extrakt aus *Radix Glycyrrhizae inflata,* enthaltend Licochalcon A.

2. Zubereitungen nach Anspruch 1, **dadurch gekennzeichnet, daß** das Gewichtsverhältnis aus Harnstoff einerseits und Licochalcon A oder einem wäßrigen Extrakt aus *Radix Glycyrrhizae inflatae,* enthaltend Licochalcon A andererseits aus dem Bereich von 100 : 1 bis 1 zu 100, bevorzugt 10 : 1 bis 1 zu 10, insbesondere bevorzugt 2 : 1 bis 1 : 2 gewählt wird.

3. Zubereitungen nach Anspruch 1 oder 2, **dadurch gekennzeichnet, daß** sie 0,01 bis 60 Gew.-%, bevorzugt 0,1 bis 25 Gew.-%, insbesondere bevorzugt 1 bis 10 Gew.-% an Harnstoff enthalten, bezogen auf das Gesamtgewicht der Zubereitungen.

4. Wasser-in-Öl-Emulsionen nach einem der vorstehenden Ansprüche, **dadurch gekennzeichnet, daß** sie enthaltend 0,001 - 10 Gew.-%, besonders bevorzugt 0,01 - 1 Gew.-%, an Licochalcon A, bezogen auf die Gesamtzusammensetzung der Zubereitung, enthalten.

5. Wasser-in-Öl-Emulsionen nach einem der vorstehenden Ansprüche, **dadurch gekennzeichnet, daß** sie als Lotionen vorliegen, welche bei 25 °C Viskositäten von 2.000 - 10.000 mPas (= Millipascalsekunden) aufweisen, ermittelt bei Scherraten von 10 Pa/s.
